(19) Europäisches Patentamt
European Patent Office
Office européen des brevets

(11) EP 0 651 757 B1

# (12) EUROPÄISCHE PATENTSCHRIFT

(45) Veröffentlichungstag und Bekanntmachung des Hinweises auf die Patenterteilung:
**29.01.1997 Patentblatt 1997/05**

(21) Anmeldenummer: **93915895.2**

(22) Anmeldetag: **13.07.1993**

(51) Int Cl.[6]: **C07F 9/38**, A61K 31/66

(86) Internationale Anmeldenummer:
**PCT/EP93/01833**

(87) Internationale Veröffentlichungsnummer:
**WO 94/02492 (03.02.1994 Gazette 1994/04)**

## (54) NEUE ACYCLISCHE AMIDINGRUPPEN-HALTIGE DIPHOSPHONSÄUREDERIVATE, VERFAHREN ZU DEREN HERSTELLUNG UND DIESE VERBINDUNGEN ENTHALTENDE ARZNEIMITTEL

NOVEL ACYCLIC AMIDINE-GROUP-CONTAINING DIPHOSPHONIC ACID DERIVATIVES, PROCESS FOR PRODUCING THEM AND MEDICAMENTS CONTAINING THESE COMPOUNDS

NOUVEAUX DERIVES ACYCLIQUES D'ACIDE DIPHOSPHONIQUE CONTENANT DES GROUPES AMIDINE, LEUR PROCEDE DE FABRICATION ET MEDICAMENTS RENFERMANT CES COMPOSES

(84) Benannte Vertragsstaaten:
**AT BE CH DE DK ES FR GB GR IE IT LI LU NL PT SE**

(30) Priorität: **21.07.1992 DE 4223940**

(43) Veröffentlichungstag der Anmeldung:
**10.05.1995 Patentblatt 1995/19**

(73) Patentinhaber: **BOEHRINGER MANNHEIM GMBH**
**68298 Mannheim (DE)**

(72) Erfinder:
- **ZILCH, Harald**
  **D-68305 Mannheim (DE)**
- **BAUSS, Frieder**
  **D-67245 Lambsheim (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 651 757 B1

**Beschreibung**

Die vorliegende Erfindung betrifft neue Amidingruppen-haltige Diphosphonsäurederivate, Verfahren zu deren Herstellung sowie Arzneimittel, die diese Substanzen enthalten.

In der DE 18 13 659 sind Diphosphonsäurederivate beschrieben, von denen die 1-Hydroxyethan-1,1-diphosphonsäure als Mittel zur Behandlung von Morbus Paget Bedeutung erlangt hat.

Das EP-A-282 320 beschreibt substituierte 3-Isoxazolylaminomethylendiphosphonsäuren und deren Ester mit antihypercalcämischer und antiarthritischer Wirkung.

In der EP-A-282 309 sind "Azol"-aminomethylendiphosphonsäuren als Hypercalcämie-Inhibitoren beschrieben.

Weiterhin kennt man aus JP-A-63/150 290 Aminomethylendiphosphonsäuren als Regulatoren des Calcium-Metabolismus und aus EP-A-274 158 Tetrahydropyrimidinyl- und Tetrahydropyridylaminomethylendiphosphonsäuren zur Behandlung eines abnormen Calcium- und Phosphat-Stoffwechsels.

Cyclische amidingruppen-haltige geminale Diphosphonsäuren kennt man aus der DE-A-3 208 600, Liebigs Ann. Chem. 1982, 275 und DE-A-39 30 130.3.

Es wurde nun gefunden, daß acyclische Derivate dieser Verbindungen außerordentlich gute Calcium-Komplexbildner sind, zusätzlich aber auch eine ausgezeichnete Wirkung auf den Calciumstoffwechsel zeigen und damit zur breiten Behandlung von Calciumstoffwechselstörungen geeignet sind. Sie lassen sich vor allem sehr gut dort einsetzen, wo der Knochenauf- und -abbau gestört ist, d.h. sie sind geeignet zur Behandlung von Erkrankungen des Skelettsystems wie z.B. Osteoporose, Morbus Paget, Morbus Bechterew u.a..

Aufgrund dieser Eigenschaften finden sie aber auch Verwendung in der Therapie von Knochenmetastasen, der Urolithiasis und zur Verhinderung heterotoper Ossifikationen. Durch ihre Beeinflussung des Calciumstoffwechsels bilden sie weiterhin eine Grundlage für die Behandlung der rheumatoiden Arthritis, der Osteoarthritis und der degenerativen Arthrose.

Gegenstand der vorliegenden Erfindung sind demnach Diphosphonate der allgemeinen Formel I

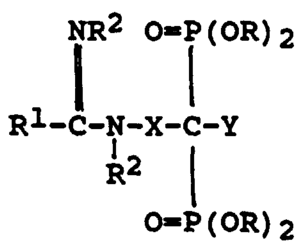

(I),

in der

- $R^1$ Wasserstoff, einen geradkettigen, verzweigten, gesättigten oder ungesättigten, ggf. durch Phenyl substituierten Alkylrest mit 1 - 9 Kohlenstoffatomen oder einen Phenylring bedeutet, der gegebenenfalls durch $C_1$-$C_3$-Alkyl, $C_1$-$C_3$-Alkoxy oder Halogen substituiert ist,
- $R^2$ Wasserstoff oder $C_1$-$C_3$-Alkyl bedeutet, wobei die beiden Reste gleich oder verschieden sein können,
- R Wasserstoff oder einen geradkettigen oder verzweigten Alkylrest mit 1 - 4 Kohlenstoffatomen darstellt,
- X eine Alkylenkette mit 1 - 6 Kohlenstoffatomen, die ein-oder mehrfach durch $C_1$-$C_3$-Alkyl-substituiert sein kann, bedeutet und gegebenenfalls durch Sauerstoff unterbrochen sein kann, wobei 1 oder 2 Kohlenstoffatome der Alkylenkette gegebenenfalls Bestandteil eines Cyclopentyl-bzw. Cyclohexylrings sein können und
- Y Wasserstoff, Hydroxy oder ggf. durch Alkylgruppe mit 1 - 6 Kohlenstoffatomen substituiertes Amino bedeuten,

sowie deren pharmakologisch unbedenkliche Salze.

Die Gruppe Y steht vorzugsweise für Wasserstoff, Hydroxy oder Amino, das durch Methyl-, Ethyl- oder Isopropyl substituiert sein kann.

Für die Gruppe X kommt bevorzugt eine Ethylen-, Propylen-, Butylen-, 1-Methylpropylen-, 2-Methylpropylen-, 1-Methylbutylen- und 2-Methylbutylengruppe in Frage.

Außerdem stellt X bevorzugt einen 1,1- bzw. 1,2-substituierten Cyclohexyl- oder Cyclopentylring dar, der über Methylen, Ethylen oder Propylen an den Bisphosphonsäureteil gebunden ist.

Der Rest R bedeutet vorzugsweise Wasserstoff oder den Methyl-, Ethyl- oder Isobutylrest.

Besonders bevorzugt sind Verbindungen, in denen R und $R^2$ gleich Wasserstoff ist, Y Wasserstoff oder Hydroxy bedeutet und $R^1$ Wasserstoff oder einen Alkylrest darstellt.

X steht besonders bevorzugt für Methylen, Ethylen, Propylen oder Butylen.

Die Verbindungen können als Stereoisomerengemisch oder als reine cis- bzw. trans-Isomere vorliegen.

Asymmetrische Kohlenstoffatome können die R- bzw. S-Konfiguration besitzen, und die Verbindungen können optisch aktiv oder als Racemate vorliegen.

Die Verbindungen der allg. Formel I werden nach an sich bekannten Verfahren hergestellt, vorzugsweise indem man eine Carbonsäure der allgemeinen Formel II

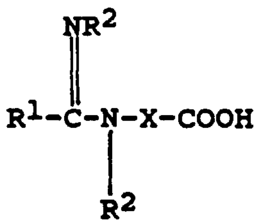

(II),

in der $R^1$, $R^2$ und X die oben angegebene Bedeutung haben, mit einem Gemisch aus phosphoriger Säure oder Phosphorsäure und einem Phosphorhalogenid oder Phosphorylhalogenid umsetzt, oder das Phosphorhalogenid auch allein in Gegenwart von Wasser zur Reaktion bringen kann, und anschließend zur freien Diphosphonsäure hydrolysiert, oder gewünschtenfalls die isolierten Verbindungen der allgemeinen Formel I in deren Ester bzw. in pharmakologisch unbedenkliche Salze überführt.

Die Carbonsäuren der Formel II sind neu und lassen sich darstellen, indem man a) Acrylester oder substituierten Acrylester mit Amindinen der allgemeinen Formel III

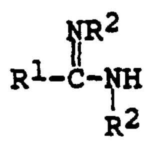

(III)

umsetzt und die Ester zu den entsprechenden freien Carbonsäuren hydrolysiert, b) Pyrimidinone der allgemeinen Formel IV

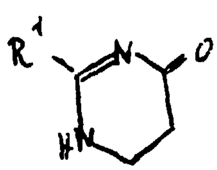

(IV)

mit Alkali- oder Erdalkalihydroxiden öffnet und in die freie Säure überführt, c) Nitrile der Formel V

$$Ne\equiv C\text{-}X\text{-}COOR^E \qquad (V)$$

mit Aminen zur Reaktion bringt und die Estergruppe $R^E$ verseift, oder d) eine aktivierte Verbindung der Formel VI

$$Z\text{-}X\text{-}COOR^E \qquad (VI),$$

in der Z z. B. ein Halogenatom darstellt, mit einem Amidin der Formel III umsetzt und nach Esterhydrolyse die freie Säure erhält.

Die bei dem Herstellungsverfahren eingesetzten Carbonsäuren der allg. Formel II werden mit 1-5, vorzugsweise 2- 3 mol phosphoriger Säure oder Phosphorsäure und 1-5, vorzugsweise 2-3 mol Phosphorylhalogenid, Phosphortrihalogenid oder Phosphorpentahalogenid versetzt und bei 80-130°C, vorzugsweise 100°C zur Reaktion gebracht. Bei den Phosphor- oder Phosphorylhalogeniden handelt es sich vorzugsweise um die Chloride bzw. Bromide. Man kann die Reaktion auch in Gegenwart von Verdünnungsmitteln wie Halogenkohlenwasserstoffen, insbesondere Chlorbenzol, Tetrachlorethan oder auch Dioxan, gegebenenfalls unter Zusatz von Wasser, durchführen. Die anschließende Hydrolyse erfolgt durch Erhitzen mit Wasser, zweckmäßiger jedoch mit halbkonzentrierter Salz- oder Bromwasserstoffsäure.

Die freien Diphosphonsäuren der allgemeinen Formel I können durch Erhitzen mit Orthoameisensäurealkylestern in die entsprechenden Tetraalkylester überführt werden und zu Diestern oder wieder zu den freien Tetrasäuren verseift werden. Die Verseifung zu Diestern geschieht in der Regel dadurch, daß man den Tetraalkylester mit einem Alkalihalogenid, vorzugsweise Natriumjodid in einem geeigneten Lösungsmittel wie z.B. Aceton bei Zimmertemperatur behandelt.

Hierbei entsteht das symmetrische Diester/Dinatriumsalz, das gegebenenfalls durch einen sauren Ionenaustauscher in die Diester/Disäure umgewandelt werden kann. Die Verseifung der Ester zu freien Diphosphonsäuren geschieht in der Regel durch Kochen mit Salz- oder Bromwasserstoffsäure. Man kann jedoch auch eine Spaltung mit Trimethylsilylhalogenid, vorzugsweise dem Bromid oder Jodid vornehmen.

Als pharmakologisch verträgliche Salze werden vor allem Mono- bzw. Dialkali- oder Ammoniumsalze verwendet, die in üblicher Weise z. B. durch Titration der Verbindungen mit anorganischen oder organischen Basen wie z.B. Natrium- oder Kaliumhydrogencarbonat, Natronlauge, Kalilauge, wässrigem Ammoniak oder Aminen wie z.B. Trimethyl-, Triethyl- oder Cyclohexylamin herstellt werden. Weiterhin sind Calcium-, Zink- und Magnesiumsalze von besonderer Bedeutung.

Die Salze werden in der Regel durch Umfällen aus Wasser/Methanol oder Wasser/Aceton gereinigt.

Die erfindungsgemäßen neuen Substanzen der Formel I und ihre Salze können in flüssiger oder fester Form enteral oder parenteral appliziert werden. Hierbei kommen alle üblichen Applikationsformen infrage, beispielsweise Tabletten, Kapseln, Dragees, Sirupe, Lösungen, Suspensionen etc.. Als Injektionsmedium kommt vorzugsweise Wasser zur Anwendung, welches die bei Injektionslösungen üblichen Zusätze wie Stabilisierungsmittel, Lösungsvermittler und Puffer enthält.

Derartige Zusätze sind z.B. Tartrat- und Citrat-Puffer, Ethanol, Komplexbildner (wie Ethylendiamintetraessigsäure und deren nichttoxische Salze), hochmolekulare Polymere (wie flüssiges Polyethylenoxid) zur Viskositätsregelung. Flüssige Trägerstoffe für Injektionslösungen müssen steril sein und werden vorzugsweise in Ampullen abgefüllt. Feste Trägerstoffe sind z.B. Stärke, Lactose, Mannit, Methylcellulose, Talkum, hochdisperse Kieselsäuren, höhermolekulare Fettsäuren (wie Stearinsäure), Gelatine, Agar-Agar, Calciumphosphat, Magnesiumstearat, tierische und pflanzliche Fette, feste hochmolekulare Polymere (wie Polyethylenglykole); für orale Applikation geeignete Zubereitungen können gewünschtenfalls Geschmacks- und Süßstoffe enthalten.

Die Dosierung kann von verschiedenen Faktoren, wie Applikationsweise, Spezies, Alter und/oder individuellem Zustand abhängen. Die täglich zu verabreichenden Dosen liegen bei etwa 1-1000 mg/Mensch, vorzugsweise bei 10-200 mg/Mensch und können auf einmal oder mehrere Male verteilt eingenommen werden.

Bevorzugt im Sinne der vorliegenden Erfindung sind außer den in den Beispielen genannten Verbindungen und durch Kombination aller in den Ansprüchen genannten Bedeutungen der Substituenten ableitbaren Verbindungen die folgenden Diphosphonsäuren, sowie deren Natriumsalze, Methyl- bzw. Ethylester:

Bevorzugte Verbindungen:

1) 1-Hydroxy-2-(1-iminoethyl)aminoethan-1,1-diphosphonsäure

2) 1-Hydroxy-4-(1-iminoethyl)aminobutan-1,1-diphosphonsäure

3) 1-Hydroxy-5-(1-iminoethyl)aminopentan-1,1-diphosphonsäure

4) 1-Hydroxy-3-(1-iminopropyl)aminopropan-1,1-diphosphonsäure

5) 1-Hydroxy-4-(1-iminobutyl)-aminobutan-1,1-diphosphonsäure

6) 1-Hydroxy-6-(1-iminoethyl)aminohexan-1,1-diphosphonsäure

7) 1-Hydroxy-3-(1-iminoethyl)aminobutan-1,1-diphosphonsäure

8) 1-Hydroxy-4-(1-iminoethyl)aminopentan-1,1-diphosphonsäure

9) 1-Hydroxy-5-(1-iminoethyl)aminohexan-1,1-diphosphonsäure

10) 1-Hydroxy-3-(1-iminopropyl)aminobutan-1,1-diphosphonsäure

11) 1-Hydroxy-4-(1-iminoethyl)amino-3-methylbutan-1,1-diphosphonsäure

12) 1-Hydroxy-3-(1-iminobutyl)aminopropan-1,1-diphosphonsäure

13) 1-Hydroxy-3-[1-(methylimino)ethyl]aminopropan-1,1-diphosphonsäure

14) 1-Hydroxy-3-[2-(1-iminoethyl)aminocyclohexyl]-propan-1,1-diphosphonsäure

15) 1-Hydroxy-3-[2-(1-iminoethyl)aminocyclopentyl]-propan-1,1-diphosphonsäure

16) 1-Hydroxy-3-[1-(1-iminoethyl)aminocyclohexyl]-propan-1,1-diphosphonsäure

17) 1-Hydroxy-2-[2-(1-iminopropyl)aminocyclohexyl]-ethan-1,1-diphosphonsäure

18) 1-Hydroxy-2-(iminomethyl)aminoethan-1,1-diphosphonsäure

19) 3-(Iminoethyl)aminoethan-1,1-diphosphonsäure

20) 2-(1-Iminoethyl)aminoethan-1,1-diphosphonsäure

21) 1-Hydroxy-3-(iminomethyl)aminopropan-1,1-diphosphonsäure

22) 3-(1-Iminoethyl)aminopropan-1,1-diphosphonsäure

23) 3-[2-(1-Iminoethyl)aminocyclohexyl]-propan-1,1-diphosphonsäure

24) 1-Hydroxy-3-[2-(Iminomethyl)aminocyclohexyl]-propan-1,1-diphosphonsäure

Beispiel 1

2-Methyl-5,6-dihydro-1H-pyrimidin-4-on

14 g Acetamidin-Hydrochlorid wurden in eine Lösung aus 3.4 g Natrium in 118 ml abs. Ethanol eingetragen und 30 Minuten bei Raumtemperatur gerührt.

Dann wurden innerhalb von 30 Minuten 15.4 ml Acrylsäuremethylester zugetropft und weitere 5 Stunden bei Raumtemperatur gerührt.

Nach Zugabe von 100 ml Aceton wurde der Niederschlag abgesaugt, das Filtrat im Vakuum eingedampft und der Rückstand aus Ethanol umkristallisiert. Ausbeute 13.5 g (56 % d. Th.), Schmp. 127-131° C [lt. NMR in DMSO handelt es sich um die Enolform].

3-(1-Iminoethyl)aminopropionsäure

4 g 2-Methyl-5,6-dihydro-1H-pyrimidin-4-on wurden mit 20 g $Ba(OH)_2 x 8H_2O$ in 350 ml Wasser 5 Stunden auf 50°C erhitzt.

Dann ließ man auf Raumtemperatur abkühlen, filtrierte den vorhandenen weißen Niederschlag ab und säuerte das Filtrat mit 2N $H_2SO_4$ an.

Nach 20-stündigem Stehen im Kühlschrank wurde das gefällte $BaSO_4$ abgesaugt, das Filtrat im Rotationsverdampfer eingeengt und der Rückstand aus Ethanol mit Ether gefällt. Der getrocknete Niederschlag wurde ohne weitere Reinigung in die Phosphorylierung eingesetzt.

1-Hydroxy-3-(1-iminoethyl)aminopropan-1,1-diphosphonsäure

5 g 3-(1-Iminoethyl)aminopropionsäure wurden bei 80° C mit 6.7 g $H_3PO_3$ verschmolzen, unter Rühren langsam mit 7.5 ml $POCl_3$ versetzt und 24 Stunden bei 80° C gehalten.

Dann wurde das überschüssige $POCl_3$ im Vakuum abgezogen, der Rückstand mit 80 ml Wasser versetzt und die klare Lösung für 1 Stunde auf 100° C erhitzt.

Nach dem Abkühlen wurde im Rotationsverdampfer eingeengt und durch Ionenaustauscherchromatographie an Amberlite IR 120 ($H^+$-Form) mit Wasser als Eluens gereinigt.

Die nach DC einheitlichen Fraktionen wurden vereinigt, eingedampft und aus Wasser/Aceton kristallisiert. Ausbeute 5.2 g (53 % d. Th., bezogen auf eingesetztes 2-Methyl-5,6-dihydro-1H-pyrimidin-4-on), Schmp. > 106° C Zers., $R_f$=0.36 (EtOH/$H_2O$/HOAc 9/1/1).

**Retinoid-Test**

Die Versuchsdurchführung erfolgte in Anlehnung an die Methode von Trechsel, Stutzer und Fleisch (J. Clin. Invest. 80, 1679-1686, 1987) in thyreoparathyreoidektomierten Ratten (TPTX). Die Dosisangaben für die Bisphosphonate erfolgen aus Gründen der Vergleichbarkeit mit der Literatur und mit bisher erhobenen externen Daten in mg P/kg (1 mg P/kg entspricht 16.13 μMol/kg). Bei den angegebenen Dosierungen handelt es sich immer um die Tagesdosis.

Bei der Auswertung wurde der Retinoid-induzierte Anstieg des Kalzium-Spiegels (Differenz von Tag 3 zu Tag 0) als 100% festgesetzt. Die Wirkung eines Bisphosphonates (% rel. inhibition) wurde definiert als die Hemmung des Retinoid-induzierten Kalziumanstieges durch dieses Bisphosphonat im Vergleich zum Retinoid-Effekt. Stieg der Kalzium-Spiegel unter Bisphosphonat-Applikation um den gleichen Wert an, wie nach Retinoid-Applikation alleine, so war die Wirkung des Bisphosphonates 0%; erfolgte kein Anstieg des Kalzium-Spiegels, so war die Wirkung 100%; fiel der Kalzium-Spiegel unter den Ausgangswert, so war die Wirkung größer als 100%.

Die Berechnung der Wirkung (% rel. inhibition) in diesem Assay erfolgte nach folgender Formel:

$$\text{\% rel. inhibition} = \frac{\overline{\text{delta Ca}_R} - \overline{\text{delta Ca}_{BP}}}{\overline{\text{delta Ca}_R}} \times 100$$

delta $Ca_R$: Differenz der Seriumkalzämie, die nach Retinoid-Applikation (an drei aufeinanderfolgenden Tagen) induziert wurde und deren Basalwert.

delta $Ca_{BP}$: Differenz der Seriumkalzämie, die nach Retinoid-Applikation (an drei aufeinanderfolgenden Tagen) mit entsprechender Bisphosphonat-Applikation und deren Basalwert.

Tabelle

| Verbindung | Dosis mg P/kg | Applikation | % rel. Inhibition | n |
|---|---|---|---|---|
| Bsp. 1 | 0.010 | s.c. | 57.07 | 5 |
| | 0.030 | s.c. | 103.92 | 5 |
| Pamidronate | 0.010 | s.c. | 29.12 | 5 |
| | 0.030 | s.c. | 37.52 | 5 |

Pamidronate: 1-Hydroxy-3-aminopropyl-1,1-diphosphonsäure

**Patentansprüche**

1. Verbindungen der Formel I

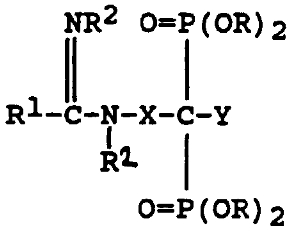

(I),

in der

$R^1$ Wasserstoff, einen geradkettigen, verzweigten, gesättigten oder ungesättigten, ggf. durch Phenyl substituierten Alkylrest mit 1 - 9 Kohlenstoffatomen oder einen Phenylring bedeutet, der gegebenenfalls durch $C_1$-$C_3$-Alkyl, $C_1$-$C_3$-Alkoxy oder Halogen substituiert ist,

$R^2$ Wasserstoff oder $C_1$-$C_3$-Alkyl bedeutet, wobei die beiden Reste gleich oder verschieden sein können,

R Wasserstoff oder einen geradkettigen oder verzweigten Alkylrest mit 1 - 4 Kohlenstoffatomen darstellt,

X eine Alkylenkette mit 1 - 6 Kohlenstoffatomen, die ein- oder mehrfach durch $C_1$-$C_3$-Alkyl-substituiert sein kann, bedeutet und gegebenenfalls durch Sauerstoff unterbrochen sein kann, wobei 1 oder 2 Kohlenstoffatome der Alkylenkette gegebenenfalls Bestandteil eines Cyclopentyl- bzw. Cyclohexylrings sein können

und

Y Wasserstoff, Hydroxy oder ggf. durch Alkylgruppe mit 1 - 6 Kohlenstoffatomen substituiertes Amino bedeuten,

sowie deren pharmakologisch unbedenkliche Salze.

**2.** Verfahren zur Herstellung von Verbindungen der Formel I

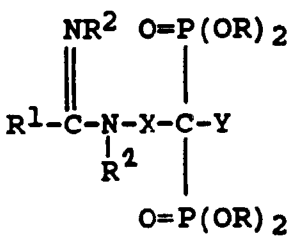

(I),

in der

$R^1$ Wasserstoff, einen geradkettigen, verzweigten, gesättigten oder ungesättigten, ggf. durch Phenyl substituierten Alkylrest mit 1 - 9 Kohlenstoffatomen oder einen Phenylring bedeutet, der gegebenenfalls durch $C_1$-$C_3$-Alkyl, $C_1$-$C_3$-Alkoxy oder Halogen substituiert ist,

$R^2$ Wasserstoff oder $C_1$-$C_3$-Alkyl bedeutet, wobei die beiden Reste gleich oder verschieden sein können,

R Wasserstoff oder einen geradkettigen oder verzweigten Alkylrest mit 1 - 4 Kohlenstoffatomen darstellt,

X eine Alkylenkette mit 1 - 6 Kohlenstoffatomen, die ein-oder mehrfach durch $C_1$-$C_3$-Alkyl-substituiert sein kann, bedeutet und gegebenenfalls durch Sauerstoff unterbrochen sein kann, wobei 1 oder 2-Kohlenstoffatome der Alkylenkette gegebenenfalls Bestandteil eines Cyclopentyl- bzw. Cyclohexylrings sein können

und

Y Wasserstoff, Hydroxy oder ggf. durch Alkylgruppe mit 1 - 6 Kohlenstoffatomen substituiertes Amino bedeuten,

sowie deren pharmakologisch unbedenkliche Salze,
dadurch gekennzeichnet, daß man eine Carbonsäure der allgemeinen Formel II

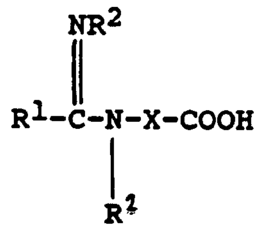

(II),

in der $R^1$, $R^2$ und X die oben angegebene Bedeutung haben, mit einem Gemisch aus phosphoriger Säure oder Phosphorsäure und einem Phosphorhalogenid oder Phosphorylhalogenid umsetzt, oder das Phosphorhalogenid auch allein in Gegenwart von Wasser zur Reaktion bringen kann, und anschließend zur freien Diphosphonsäure hydrolysiert, oder gewünschtenfalls die isolierten Verbindungen der allgemeinen Formel I in deren Ester bzw. in pharmakologisch unbedenkliche Salze überführt.

**3.** Arzneimittel, enthaltend eine Verbindung gemäß Anspruch 1 neben üblichen Träger- und Hilfsstoffen.

**4.** Verwendung von Verbindungen gemäß Anspruch 1 zur Behandlung von Calciumstoffwechselstörungen.

## Claims

**1.** Compounds of the formula I

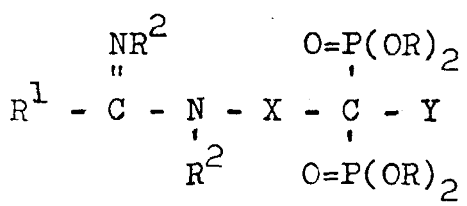

(I)

in which $R^1$ signifies hydrogen, a straight-chained branched, saturated or unsaturated alkyl radical with 1 - 9 carbon atoms possibly substituted by phenyl or a phenyl ring which is possibly substituted by $C_1$-$C_3$-alkyl, $C_1$-$C_3$-alkoxy or halogen, $R^2$ signifies hydrogen or $C_1$-$C_3$-alkyl, whereby the two radicals can be the same or different, R represents hydrogen or a straight-chained or branched alkyl radical with 1 - 4 carbon atoms, X signifies an alkylene chain with 1 - 6 carbon atoms which can be substituted one or more times by $C_1$-$C_3$-alkyl and can possibly be interrupted by oxygen, whereby 1 or 2 carbon atoms of the alkylene chain can possibly be part of a cyclopentyl or cyclohexyl ring and Y signifies hydrogen, hydroxyl or amino possibly substituted by alkyl groups with 1 - 6 carbon atoms, as well as their pharmacologically acceptable salts.

**2.** Process for the preparation of compounds of the formula I

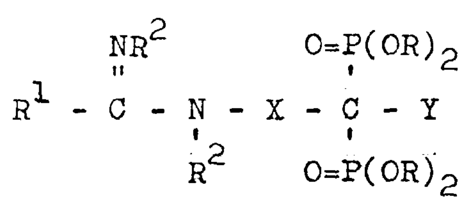

(I)

in which $R^1$ signifies hydrogen, a straight-chained, branched, saturated or unsaturated alkyl radical with 1 - 9

carbon atoms possibly substituted by phenyl or a phenyl ring which is possibly substituted by $C_1$-$C_3$-alkyl, $C_1$-$C_3$-alkoxy or halogen, $R^2$ signifies hydrogen or $C_1$-$C_3$-alkyl, whereby the two radicals can be the same or different, R represents hydrogen or a straight-chained or branched alkyl radical with 1 - 4 carbon atoms, X signifies an alkylene chain with 1 - 6 carbon atoms which can be substituted one or more times by $C_1$-$C_3$-alkyl and can possibly be interrupted by oxygen, whereby 1 or 2 carbon atoms of the alkylene chain can possibly be part of a cyclopentyl or cyclohexyl ring and Y signifies hydrogen, hydroxyl or amino possibly substituted by alkyl groups with 1 - 6 carbon atoms, as well as of their pharmacologically acceptable salts, characterised in that one reacts a carboxylic acid of the general formula II

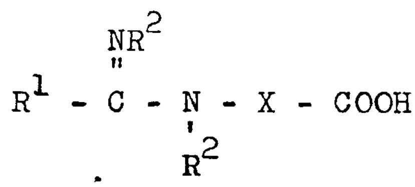 (II)

in which $R^1$, $R^2$ and X have the above-given meaning, with a mixture of phosphorous acid or phosphoric acid and a phosphorus halide or phosphoryl halide or can also bring to reaction the phosphorus halide alone in the presence of water and subsequently hydrolyses to the free diphosphonic acid or, if desired, converts the isolated compounds of the general formula I into their esters or into pharmacologically acceptable salts.

**3.** Medicaments containing a compound according to claim 1, besides usual carrier and adjuvant materials.

**4.** Use of compounds according to claim 1 for the treatment of calcium metabolism disturbances.

**Revendications**

**1.** Composés de formule I

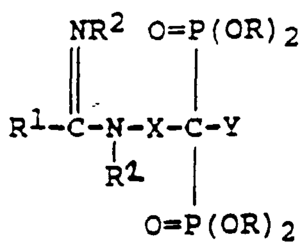 (I),

dans laquelle

$R^1$ représente un atome d'hydrogène, un reste alkyle saturé ou insaturé, à chaîne linéaire ou ramifiée, éventuellement substitué par un groupe phényle, ayant 1 à 9 atomes de carbone ou un noyau phényle qui est éventuellement substitué par un groupe alkyle en $C_1$-$C_3$, alcoxy en $C_1$-$C_3$ ou halogéno,

$R^2$ représente un atome d'hydrogène, un groupe alkyle en $C_1$-$C_3$, les deux restes pouvant être identiques ou différents,

R représente un atome d'hydrogène ou un reste alkyle à chaîne linéaire ou ramifiée, ayant 1 à 4 atomes de carbone,

X représente une chaîne alkylène ayant 1 à 6 atomes de carbone, qui peut être substituée une ou plusieurs fois par un groupe alkyle en $C_1$-$C_3$ et qui peut être éventuellement interrompue par un atome d'oxygène, 1 ou 2 atomes de carbone de la chaîne alkylène pouvant faire partie éventuellement d'un cycle cyclopentyle ou cyclohexyle

et

Y représente un atome d'hydrogène, un groupe hydroxy ou amino éventuellement substitué par un groupe alkyle ayant 1 à 6 atomes de carbone,

ainsi que leurs sels pharmacologiquement acceptables.

**2.** Procédé de préparation de composés de formule I

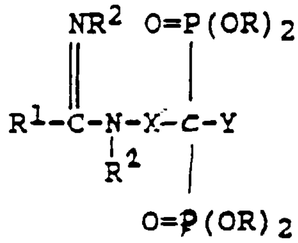

(I),

dans laquelle

$R^1$ représente un atome d'hydrogène, un reste alkyle saturé ou insaturé, à chaîne linéaire ou ramifiée, éventuellement substitué par un groupe phényle, ayant 1 à 9 atomes de carbone ou un noyau phényle qui est éventuellement substitué par un groupe alkyle en $C_1$-$C_3$, alcoxy en $C_1$-$C_3$ ou halogéno,

$R^2$ représente un atome d'hydrogène, un groupe alkyle en $C_1$-$C_3$, les deux restes pouvant être identiques ou différents,

R représente un atome d'hydrogène ou un reste alkyle à chaîne linéaire ou ramifiée, ayant 1 à 4 atomes de carbone,

X représente une chaîne alkylène ayant 1 à 6 atomes de carbone, qui peut être substituée une ou plusieurs fois par un groupe alkyle en $C_1$-$C_3$ et qui peut être éventuellement interrompue par un atome d'oxygène, 1 ou 2 atomes de carbone de la chaîne alkylène pouvant faire partie éventuellement d'un cycle cyclopentyle ou cyclohexyle

et

Y représente un atome d'hydrogène, un groupe hydroxy ou amino éventuellement substitué par un groupe alkyle ayant 1 à 6 atomes de carbone,

ainsi que leurs sels pharmacologiquement acceptables,
caractérisé en ce que l'on fait réagir un acide carboxylique de formule générale II

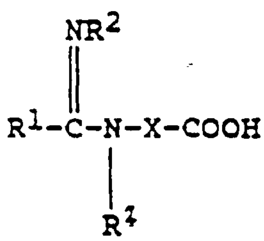

(II),

dans laquelle $R^1$, $R^2$ et X ont les significations indiquées ci-dessus, avec un mélange d'acide phosphoreux ou

phosphorique et d'halogénure de phosphore ou d'halogénure de phosphoryle, ou on peut également amener à réagir l'halogénure de phosphore seul en présence d'eau et ensuite, hydrolyser le produit formé en acide diphosphonique libre, ou éventuellement, on transforme les composés isolés, de formule générale I, en leur ester ou sel pharmacologiquement acceptable.

**3.** Médicament, contenant un composé selon la revendication 1 en plus de véhicules et adjuvants usuels.

**4.** Utilisation de composés selon la revendication 1 dans le traitement de troubles du métabolisme calcique.